Europäisches Patentamt

European Patent Office

Office européen des brevets

(19)

(11) Publication number: **0 193 716**
A2

(12)

# EUROPEAN PATENT APPLICATION

(21) Application number: 86100606.2

(22) Date of filing: 17.01.86

(51) Int. Cl.⁴: **B 01 D 53/04,** C 01 B 31/20, C 07 C 9/04

(30) Priority: 25.01.85 US 695151

(43) Date of publication of application: 10.09.86 Bulletin 86/37

(84) Designated Contracting States: DE FR GB

(71) Applicant: **AIR PRODUCTS AND CHEMICALS, INC., P.O. Box 538, Allentown, Pennsylvania 18105 (US)**

(72) Inventor: **Sircar, Shivaji, 1508 Bogie Avenue, Wescosville PA 18106 (US)**
Inventor: **Koch, William Rohrer, RD 1 Box 1216, Fleetwood PA (US)**

(74) Representative: **Kador & Partner, Corneliusstrasse 15, D-8000 München 5 (DE)**

(54) Adsorptive separation of methane and carbon dioxide gas mixtures.

(57) A process is described for separating gas mixtures containing a primary gaseous component and a secondary gaseous component by selective adsorption of the secondary gaseous component in an adsorptive process including the steps of adsorption in at least one adsorbent bed, rinsing said bed with secondary component, depressurizing and evacuating said bed without addition rinsing and repressurizing said bed with primary gaseous component. The process is particularly attractive for recovering carbon dioxide and methane from landfill gas.

211-P-US03169

# ADSORPTIVE SEPARATION OF METHANE AND CARBON DIOXIDE GAS MIXTURES

## TECHNICAL FIELD

The present invention is directed to the separation of gaseous mixtures in an adsorptive separation utilizing a pressure swing cycle. More particularly, the present invention is directed to the separation of methane/carbon dioxide gas mixtures such as are recovered in enhanced oil recovery and landfill gas recovery operations.

## BACKGROUND OF THE PRIOR ART

Adsorptive separations using pressure swing cyclic procedures for recovering or isolating components of gas mixtures are well known in the art. These systems typically utilize a series of parallel adsorptive beds operated in a switching pattern wherein at least one bed is in an adsorption sequence while another bed is in a regeneration sequence. Generally recognized steps in adsorptive separation include adsorption, depressurization, evacuation, purging and repressurization combined in various sequential arrangements and not necessarily in that order.

For instance, in U.S. Patent 3,176,444 a pressure swing adsorption process is disclosed wherein a feed gas is separated into a product and impurities which are adsorbed on the adsorbent in the various beds of the process. It is recited that adsorption is continued only so long as the adsorption front is still within the bed after which depressurization is performed cocurrently to utilize the rest of the adsorbent bed before additional desorbing to remove impurities from the bed is performed for the purpose of regeneration.

In another adsorptive scheme, U.S. Patent 3,226,913 discloses a separation process using pressure swings wherein the adsorption is terminated short of breakthrough of the bed and three stages of sequential depressurization are utilized in conjunction with a purge gas which constitutes preferably a separate purge medium.

U.S. Patent 3,430,418 provides an adsorptive separation wherein adsorption is conducted short of full use of the adsorption bed, cocurrent depressurization is performed wherein depressurized gas is used immediately to repressurize a second bed and finally additional steps of depressurization are performed to regenerate the bed.

U.S. Patent 3,751,878 discloses a process for the separation of methane from carbon dioxide in a feed gas mixture using an adsorptive separation technique including an adsorption step, a carbon dioxide rinse step and a pressure reduction step.

In U.S. Patent 3,977,845 an adsorptive separation technique is disclosed using an additional segregated adsorptive bed to recover a product gas and a low purity gas from a feed gas mixture.

U.S. Patent 4,021,210 provides an adsorptive process using an adsorptive step, two depressurization steps and a purge step to remove residual components from the adsorptive media.

U.S. Patent 4,000,990 discloses an adsorptive separation of landfill gas using three or more adsorptive beds and a pretreatment zone. The process uses double bed adsorption zones and doesn't rinse between adsorption and venting.

A relevant separation of hydrogen and carbon dioxide or methane and carbon dioxide is disclosed in U.S. Patent 4,077,779 to a common assignee wherein an adsorptive separation is carried out with a series of parallel adsorptive beds of 4, 5 and 6 beds in which the following steps are performed: an adsorption step, a rinse step with secondary component, a depressurization step, an inert or air purge step, an evacuation step and a repressurization step. In order to practice the separation technique of this patent, it is necessary to perform two purges, one involving secondary product which is recovered from the gas mixture being separated and another purge that involves the use of an inert gas or an air purge which requires an outside gas source to be utilized and results in at least a temporary contamination of the regenerating bed being purged. This has been shown to create a loss in secondary product recovery. The separatory apparatus thus has a disadvantage when the desired goal is the attainment of high recoveries of both primary and secondary product such as in the separation and recovery of methane/carbon dioxide mixtures.

The present invention which deletes the use of an air or inert gas purge in an adsorptive process similar to U.S. 4,077,779 provides an unexpected enhancement of the secondary product recovery (carbon dioxide) which is particularly amenable to processes such as certain processes for enhanced recovery of petroleum or landfill gas applications wherein the carbon dioxide is a potential product or could be utilized for pressure enhancement. The present invention also constitutes an improvement over this prior art in capital costs of an installation while offering process simplicity by the reduction in the number of steps and the amount of gaseous materials necessary to operate the process. The problems attendant with the prior art, particularly of low recovery of secondary component of a gas mixture in an adsorptive separation, are overcome by the present invention which will be described in greater detail below.

## BRIEF SUMMARY OF THE INVENTION

The present invention is directed to a process for the adsorptive separation of a gas mixture containing at least a primary gaseous component and a secondary gaseous component in which each component is recovered at high recovery and high purity in a plurality of adsorptive beds which are preferentially selective to the adsorption of the secondary component comprising the steps of: passing a feed gas mixture into one of the plurality of adsorption beds wherein primary component passes through the bed as a pure product and secondary component is selectively adsorbed on the adsorbent, discontinuing adsorption and rinsing the adsorption bed with a stream of secondary component to remove any primary component as an effluent which is recycled to the feed to the process, depressurizing the rinsed adsorption bed to an intermediate pressure countercurrent to said adsorption to at least partially remove the secondary component from said bed, following said depressurization and without further rinsing, evacuating said adsorption bed countercurrently to a subatmospheric pressure to further remove secondary component from said bed and repressurizing the bed to superatmospheric pressure to prepare the bed for another adsorption step.

Preferably, the process is practiced wherein the gas mixture to be separated comprises methane as a primary component and carbon dioxide as a secondary component. Such a gas mixture is typically recovered from landfill gas and produced gas from oil fields that are being oxygen fireflooded or carbon dioxide flooded. Alternately, the primary component can be hydrogen and the secondary component, carbon dioxide.

The process can be practiced preferably in a 4, 5 or 6 parallel bed configuration.

The process provides for recovery of both primary and secondary component at 95% or above. Preferably, the process is operated to recover 98% or better of both the primary and secondary component with a purity of 98% or better for both the primary and secondary component.

Optimally, a molecular sieve material or activated carbon is used as the adsorbent in the adsorptive beds of the process.

Preferably, the feed gas mixture is at a pressure in the range of 50-500 psig, optimally approximately 150 psia.

The feed gas mixture is preferably pretreated to remove water, heavy hydrocarbons and other trace impurities prior to separation of the primary and secondary components in the adsorptive beds.

Optimally, the rinse of the adsorptive bed with secondary component is performed cocurrently to the adsorption step of the process at approximately adsorption pressure. Additionally, the repressurization of the bed is performed countercurrently to the adsorption of the same bed.

## BRIEF DESCRIPTION OF THE DRAWINGS

FIG 1 is a schematic flow diagram of one embodiment of the invention using 4 beds of adsorption material to perform the separation of the present invention.

FIG 2 is a schematic flow diagram of a 5 bed embodiment of the present invention.

FIG 3 is a schematic flow diagram of a 6 bed configuration of the adsorptive separation process of the present invention.

## DETAILED DESCRIPTION OF THE INVENTION

Upgrading of low BTU gas streams is becoming increasingly important with the advent of various production assisted recovery schemes. Such schemes include the recovery of natural gas or methane from landfill sites as well as from the gases obtained during the enhanced recovery of non-naturally producing petroleum fields such as $CO_2$ flood operations and oxygen fireflood. Additionally, some industrially produced hydrogen streams such as steam reformer off-gas also contain carbon dioxide as a bulk impurity which requires upgrading for commercial application. Typically, such low BTU streams can include approximately 50% methane and 50% carbon dioxide, although the contents can vary considerably and other impurities will be present depending upon the site conditions and the particular source of the low BTU fuel gas resource. Methane and carbon dioxide mixtures offer an attractive commodity for upgrading, but require the performance of a difficult separation. In known prior art cryogenic techniques, separation of methane and carbon dioxide is extremely difficult due to the close relative volatility. Such prior art cryogenic techniques have typically utilized additional additives or solvent extractants in order to enhance the separation of the two components under cryogenic conditions. The steam-methane reformer off-gases can typically contain up to 30% carbon dioxide in hydrogen. In known techniques, hydrogen recoveries have generally been only in the 65 to 85% range.

The present invention uses a non-cryogenic adsorptive separation wherein a marketable high purity methane or hydrogen primary component is recovered in good quantity and a high purity carbon dioxide by-product or secondary component is recovered in good quantity with potential for use or venting depending upon the economic conditions at the site of the gas processing. The key to the high recovery of the methane or hydrogen product and the high purity of the carbon dioxide of the secondary product is the high pressure carbon dioxide rinse step of the adsorptive process. The key to the increased recovery of high purity carbon dioxide product in the present invention over that of prior U.S. Patent 4,077,779 is the elimination of the inert or air purge required and taught by that patent. The invention utilizes packed beds of molecular sieve or activated carbon material which is selective to the adsorption of carbon dioxide in relation to the methane or hydrogen of the feed gas mixture. In this manner, the primary component is allowed to pass through the various adsorptive beds while the secondary component, carbon dioxide, is adsorbed onto the molecular sieve adsorbent of the various beds. Adsorbents which may be utilized in the process include various cation-exchanged A or X zeolites, mordenites and various activated carbons. The feed gas composition will typically range in content of 30 to 70% methane and 70 to 30% carbon dioxide or 40 to 90% hydrogen and 60 to 10% carbon dioxide. The pressure of the feed gas is typically in the range of 50 to 500 psia, optimally approximately 150 psig.

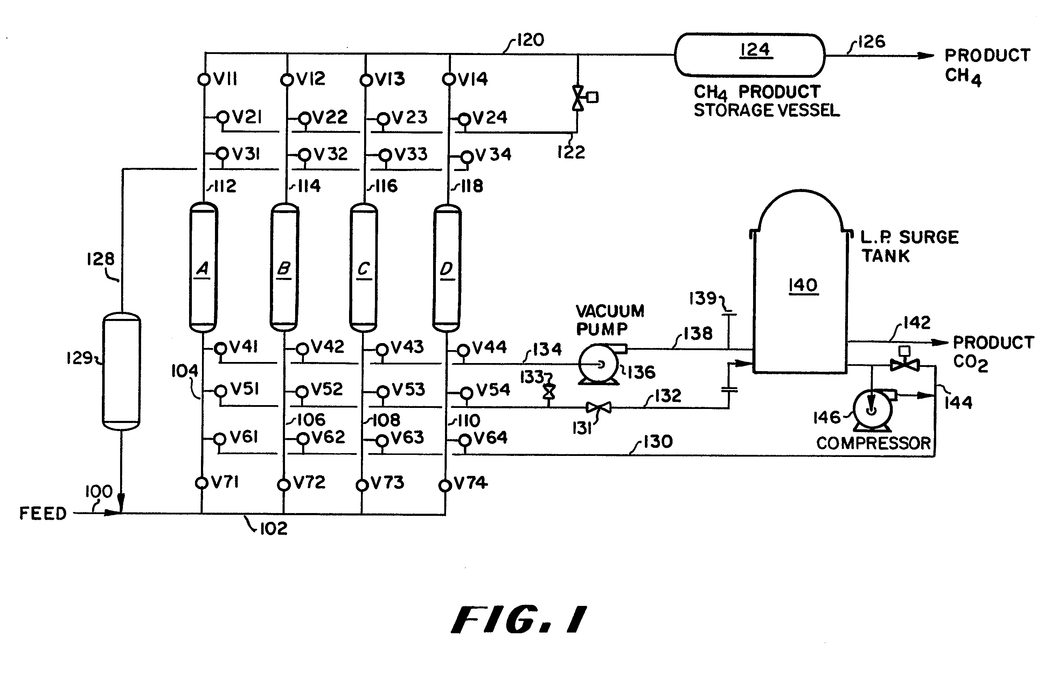

The present invention will now be described with reference to several preferred embodiments. The first embodiment constitutes a 4 bed configuration of the present invention which is illustrated in FIG 1. With reference to FIG 1 a raw landfill gas is collected and compressed to 50-500 psig via a feed compressor after which the gas stream is then cooled to near ambient temperature in a cooler condenser and any condensate is removed as a liquid in a knock-out drum. The raw gas is then treated for removal of trace chemical impurities and dried to a dew point of approximately -30° to -70°F in a pretreatment system. None of this apparatus is shown in the drawings, but such pretreatment is typical for such adsorptive systems as well documented in the prior art. The pretreated gas is then conducted through the adsorptive system of FIG 1 in a 5 step process including: adsorption, high pressure rinse, desorption, evacuation and repressurization. The pretreated gas in line 100 is first combined with a recycle gas from vessel 129 before being introduced into line 102 and sent to any one of four beds, A, B, C or D. It is understood that only one bed is receiving feed gas for adsorption purposes at a particular point in time in the sequence of the cyclic operation and that the other beds are undergoing various stages of regeneration or secondary component recovery.

For the present discussion, it will suffice that the feed gas is introduced through valve 71 into line 104 for adsorption in pressurized and regenerated bed A. The methane and carbon dioxide gas mixture passes through the bed wherein the methane is recovered as an effluent in line 112, while the carbon dioxide is adsorbed on the adsorbent (such as molecular sieve) packing of the bed to complete this separation. Methane continues through open valve 11 and passes through manifold 120 to a methane product storage vessel 124. Methane product can be drawn off in line 126 as a primary component product. However, a portion of the methane recovered will be needed for repressurization of the various beds during the course of the cyclic operation. When bed A approaches breakthrough, that is when carbon dioxide has been adsorbed through most of the bed and is about to emanate out of the bed into line 112, the adsorption in bed A is terminated and switched to another one of the three remaining beds, such as bed B.

At this point, bed A is placed in a high pressure rinse mode wherein secondary component or carbon dioxide in the low pressure surge tank 140 is removed by compressor 146 and compressed to an elevated pressure of approximately the same pressure as the feed gas and passed through line 130 and open valve 61 wherein the carbon dioxide rinse passes cocurrently through bed A to remove any remaining methane in the form of voids gas situated in the interstitial spaces between the particles of the adsorbent as well as co-adsorbed on the adsorbent material. The rinse is performed until a pure secondary component or carbon dioxide wave front approaches the effluent or downstream end of bed A upon which the rinse is terminated. During this time, the voids gas emanating through line 112 is passed through open valve 31 and is recycled in line 128 and surge tank 129 to the feed which is being sent to one of the other parallel adsorptive beds. Vessel 129 mixes recycle gas to provide a recycle to the feed of uniform composition. Compressor recycle in line 144 allows adjustment of down stream flow.

Upon termination of the rinse step, desorption is initiated, which involves a depressurization from the feed pressure condition of a pressure in the range of approximately 50-500 psia down to an intermediate pressure, preferably atmospheric pressure of 14.7 psia.

0193716

- 8 -

This depressurization is done countercurrently in a controlled let down and removes high purity carbon dioxide through open valve 51 and line 132 connecting the depressurizing column to the low pressure surge tank 140. When the desired intermediate (ambient) pressure condition is met, the regenerating bed A is then switched to the fourth step of the cyclic process; evacuation. Evacuation is continued in a countercurrent manner such as the depressurization step preceding it. In this present invention, evacuation immediately follows depressurization without any intermediate rinse or purge step being performed. In the prior art, an inert or air purge was performed between depressurization and evacuation, but this failed to produce a good recovery of high purity secondary component for a methane/carbon dioxide mixture using commonly available adsorbent. The unexpected and high recoveries of the present invention are achieved by following the depressurization step with a desorptive evacuation step as described hereafter. Bed A is evacuated through open valve 41 countercurrently by removing carbon dioxide in line 134 with vacuum system 136 which delivers the carbon dioxide in line 138 to low pressure surge tank 140 or alternately vent 139. The pressure of the bed is reduced to a pressure in the range of approximately 1.0 to 2.5 psia (50-130 torr). This evacuated carbon dioxide is combined with the carbon dioxide obtained from the previous depressurization step in a low pressure surge tank and a portion of this gas is used for high pressure rinse of another of the set of parallel adsorptive beds, while a portion may also be removed as a high purity carbon dioxide by-product. This by-product is removed in line 142. If no by-product carbon dioxide is required, the process can be operated to obtain a higher purity carbon dioxide which thereby improves the overall process efficiency. This is accomplished by collecting only the higher purity carbon dioxide from the evacuation step in the surge tank 140 and venting the lower purity carbon dioxide produced during the desorption step.

During the countercurrent depressurization step, the high purity carbon dioxide which is desorbed as the pressure is reduced from the operating pressure of approximately 150 psia to atmospheric is vented to the atmosphere by closing valve 131 on line 132 and allowing the gas to vent through the valve 133. During the evacuation step, all of the carbon dioxide by-product is collected in the surge tank 140 for use as high pressure rinse.

The advantage of operating the process in this manner is an improvement in the carbon dioxide by-product recovery. The evacuated carbon dioxide is of higher purity than the carbon dioxide collected during desorption. Thus, by only collecting the evacuation gas, the carbon dioxide purity is increased thereby increasing the methane recovery. This is unique to the present invention and offers a definite improvement over the prior art where the carbon dioxide rinse was carried out with gas collected during the depressurization step. The evacuation gas of the prior art is contaminated with the inert gas and thereby cannot be used as rinse.

When the low pressure condition is achieved in the evacuation step of bed A, the valves are switched such that the repressurization of the bed is commenced to prepare for another adsorptive step in the cycle. The repressurization is performed by admitting methane product through line 122 and open valve 21 to countercurrently repressurize the adsorptive bed A with high pressure methane product at approximately feed pressure conditions. The countercurrent repressurization drives any residual carbon dioxide in the adsorptive bed A away from the exit end of the bed to prepare the bed for a renewed adsorption sequence. Although the sequence of operation has been described with regard to bed A individually, it is understood that each of the other beds, B, C and D are involved in a similar sequence of steps through feed lines 106, 108 and 110 and effluent lines 114, 116 and 118 to complete the cyclic operation of the process. In order to more fully comprehend the exact process, reference to the cycle sequence in Table 1 providing the time interval and the column sequence for the 4 bed configuration demonstrates the relationship between the various columns and the overall production scheme. The time sequence given in Table 1 is only exemplary of the process. Other time schedules can be contemplated.

TABLE 1:  EXAMPLE OF CYCLE SEQUENCE

| Time (Minutes) | A | B | C | D |
|---|---|---|---|---|
| 0 - 1.5 | Adsorption | Evacuate | Depressurize | $CO_2$ Rinse |
| 1.5 - 3.0 | " | Repressurize | Evacuate | " |
| 3.0 - 4.5 | $CO_2$ Rinse | Adsorption | " | Depressurize |
| 4.5 - 6.0 | " | " | Repressurize | Evacuate |
| 6.0 - 7.5 | Depressurize | $CO_2$ Rinse | Adsorption | " |
| 7.5 - 9.0 | Evacuate | " | " | Repressurize |
| 9.0 - 10.5 | " | Depressurize | $CO_2$ Rinse | Adsorption |
| 10.5 - 12.0 | Repressurize | Evacuate | " | " |

The exact valve operation for the 4 bed configuration as shown in FIG 1 is set forth in Table 2 below.  In this Table, "O" represents an open valve sequence and "X" represents a closed valve sequence.

## TABLE 2
### Valve Positions PSA-4

| | | | | Time (min.) | | | | |
|---|---|---|---|---|---|---|---|---|
| VALVE | 0-1.5 | 1.5-3 | 3-4.5 | 4.5-6.0 | 6.0-7.5 | 7.5-9 | 9-10.5 | 10.5-2. |
| 11 | 0 | 0 | X | X | X | X | X | X |
| 12 | X | X | 0 | 0 | X | X | X | X |
| 13 | X | X | X | X | 0 | 0 | X | X |
| 14 | X | X | X | X | X | X | 0 | 0 |
| 21 | X | X | X | X | X | X | X | 0 |
| 22 | X | 0 | X | X | X | X | X | X |
| 23 | X | X | X | 0 | X | X | X | X |
| 24 | X | X | X | X | X | 0 | X | X |
| 31 | X | X | 0 | 0 | X | X | X | X |
| 32 | X | X | X | X | 0 | 0 | X | X |
| 33 | X | X | X | X | X | X | 0 | 0 |
| 34 | 0 | 0 | X | X | X | X | X | X |
| 41 | X | X | X | X | X | 0 | 0 | X |
| 42 | 0 | X | X | X | X | X | X | 0 |
| 43 | X | 0 | 0 | X | X | X | X | X |
| 44 | X | X | X | 0 | 0 | X | X | X |
| 51 | X | X | X | X | 0 | X | X | X |
| 52 | X | X | X | X | X | X | 0 | X |
| 53 | 0 | X | X | X | X | X | X | X |
| 54 | X | X | 0 | X | X | X | X | X |
| 61 | X | X | 0 | 0 | X | X | X | X |
| 62 | X | X | X | X | 0 | 0 | X | X |
| 63 | X | X | X | X | X | X | 0 | 0 |
| 64 | 0 | 0 | X | X | X | X | X | X |
| 71 | 0 | 0 | X | X | X | X | X | X |
| 72 | X | X | 0 | 0 | X | X | X | X |
| 73 | X | X | X | X | 0 | 0 | X | X |
| 74 | X | X | X | X | X | X | 0 | 0 |

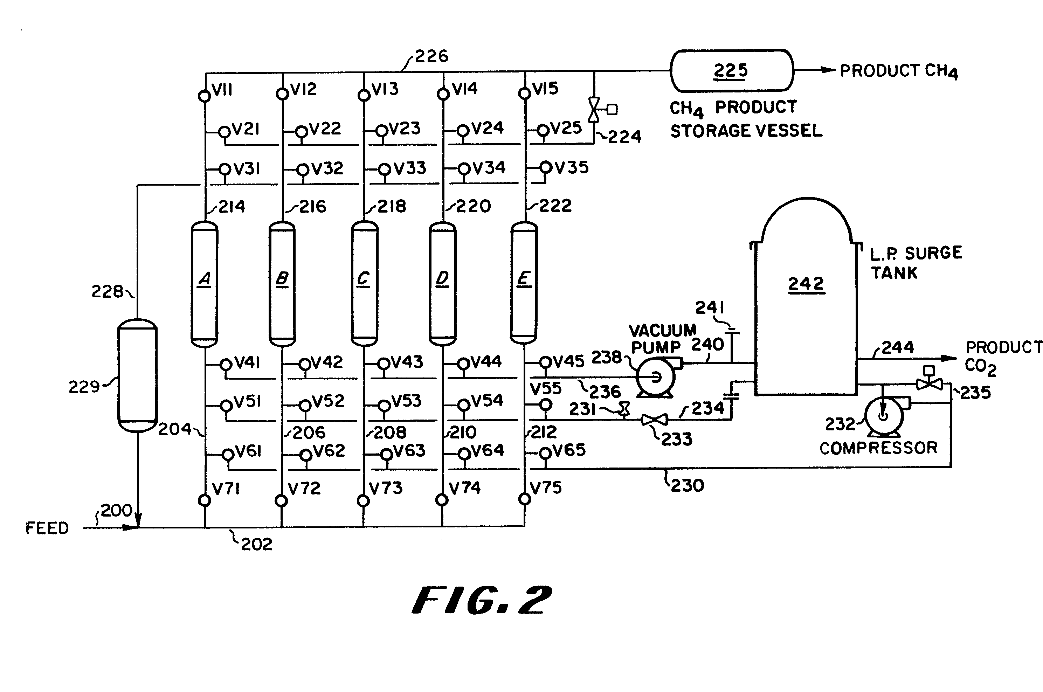

Referring to FIG 2, a 5 bed configuration of the process of the present invention is illustrated. Pretreated feed as described above is introduced in line 200 at a pressure in the range of approximately 50-500 psia, optimally 150 psia. It is combined with a recycle gas from a rinsing bed in line 228 and vessel 229. The combined gas in manifold 202 is introduced into any one of the 5 beds, A, B, C, D and E. For purposes of this discussion, the description of the sequences in bed A will be set forth. In this case, adsorption is performed by passing the gas through line 204 into bed A and evolving a high purity methane effluent in line 214. It is understood that beds B, C, D and E operate through lines 206, 208, 210, 212, 216, 218, 220 and 222 in a similar manner. The feed gas passes through open valve 71 and produces a methane product through open valve 11. This gas is then collected and removed as product in line 226 to the methane product storage vessel 225. Methane can be withdrawn as a primary component product. A portion of the gas in line 226 or vessel 225 is used for the repressurization step. When the carbon dioxide, which is selectively adsorbed on an adsorbent component in bed A reaches its saturation with the wave front near the downstream end of bed A, the adsorption step is terminated.

Bed A is then cocurrently purged with a high pressure carbon dioxide rinse gas at the feed pressure in the range of 50-500 psia. High purity carbon dioxide is removed from the surge tank 242 and compressed by the carbon dioxide rinse compressor 232 to an elevated pressure of approximately the same pressure as the feed gas. The gas is passed through line 230 and open valve 61 wherein the carbon dioxide rinse passes concurrently through bed A to remove any remaining methane in the form of void gas or co-adsorbed gas on the adsorbent. The rinse step is continued until any residual methane has been removed from bed A and the carbon dioxide is approximately at breakthrough at the downstream end of bed A. The removed void gas and co-adsorbed methane gas containing a mixture of methane and carbon dioxide of similar composition to the feed gas pass through open valve 31 and are recycled in line 228 and vessel 229 to be blended with the feed in line 200. This gas varies in purity as it comes off of bed A and the mixing vessel 229 can be used to blend the recycle to the feed line 200. When the carbon dioxide is approximately at the breakthrough point in bed A, the rinse step is terminated. Bed A is now saturated with high pressure carbon dioxide and is ready for the desorption and depressurization step. With the appropriate change in valves, the carbon dioxide is desorbed by depressurization countercurrently in bed A through open valve 51. The gas is removed in line 234 through open valve 233 and is collected in the surge tank 242. When the desorptive depressurization reaches the desired intermediate (atmospheric) pressure, Bed A is then switched to the evacuation sequence.

Again, as in the 4 bed configuration, no inert gas or air purge is performed between depressurization and evacuation as in the prior art. This achieves a higher recovery of the secondary component or carbon dioxide gas. The evacuation is initiated immediately after the desorptive depressurization step and is achieved by evacuating bed A countercurrently through open valve 41 in line 236 wherein the vacuum system 238 delivers the evacuated low pressure carbon dioxide through line 240 to the surge tank 242. The evacuated and depressurized carbon dioxide gas phases which are combined in the surge tank constitute the by-product which can be used for rinse and by-product recovered through line 244. Again, the evacuation is conducted until a desired subatmospheric pressure is reached preferably in the range of 1-2.5 psia or approximately 50-130 torr.

It is also possible to operate the five bed system by collecting only the evacuated carbon dioxide and venting through vent 231 that gas which is produced during the desorption step. As in the four bed system this will result in a higher purity carbon dioxide byproduct which in turn will improve the methane recovery. Alternately, some venting can occur through vent 241.

After the low pressure evacuation is terminated, repressurization is performed in bed A to bring it back to feed condition. Repressurization is done countercurrently by admitting some of the product from one of the other beds through open valve 21 and line 224 to deliver high pressure methane at feed pressure conditions into bed A until the pressure of the bed approximates feed conditions in the range of 50-500 psia. This drives any residual carbon dioxide to the front end of the bed and places the bed in condition for a renewed adsorption sequence. Each of the other beds, B, C, D and E goes through a similar cycle and in operating in conjunction with one another provide a continuous product flow of methane and a by-product flow of carbon dioxide. Cycle times for the 5 bed configuration are slightly different than the 4 bed configuration as one would presume and these cycle times are identified at the top of
Table 3 below.  Table 3 additionally shows the valve position for the
various valves of FIG 2 throughout the cycle sequence.  At the bottom of
Table 3 a typical representation of the sequence for bed A is set forth.
Again this process is capable of producing high purity methane at high
recovery as well as high purity carbon dioxide at high recovery wherein
the purity of the carbon dioxide is 98%+ and the recovery of the carbon
dioxide is 98%+.  The prior art, such as in U.S. Patent 4,077,779 was
unable to achieve such a high recovery of carbon dioxide.

## TABLE 3
### Valve Positions PSA-5

| Valve | Time (Min.) | | | | |
| | 1-3 | 3-6 | 6-9 | 9-12 | 12-15 |
|---|---|---|---|---|---|
| 11 | O | X | X | X | X |
| 12 | X | X | X | X | O |
| 13 | X | X | X | O | X |
| 14 | X | X | O | X | X |
| 15 | X | O | X | X | X |
| 21 | X | X | X | X | O |
| 22 | X | X | X | O | X |
| 23 | X | X | O | X | X |
| 24 | X | O | X | X | X |
| 25 | O | X | .X | X | X |
| 31 | X | O | X | X | X |
| 32 | O | X | X | X | X |
| 33 | X | X | X | X | O |
| 34 | X | X | X | O | X |
| 35 | X | X | O | X | X |
| 41 | X | X | X | O | X |
| 42 | X | X | O | X | X |
| 43 | X | O | X | X | X |
| 44 | O | X | X | X | X |
| 45 | X | X | X | X | O |
| 51 | X | X | O | X | X |
| 52 | X | O | X | X | X |
| 53. | O | X | X | X | X |
| 54 | X | X | X | X | O |
| 55 | X | X | X | O | X |
| 61 | X | O | X | X | X |
| 62 | O | X | X | X | X |
| 63 | X | X | X | X | O |
| 64 | X | X | X | O | X |
| 65 | X | X | O | X | X |
| 71 | O | X | X | X | X |
| 72 | X | X | X | X | O |
| 73 | X | X | X | O | X |
| 74 | X | X | O | X | X |
| 75 | X | O | X | X | X |
| Bed A → | Adsorption | H.P. Rinse | Depress | Evac | Repress |

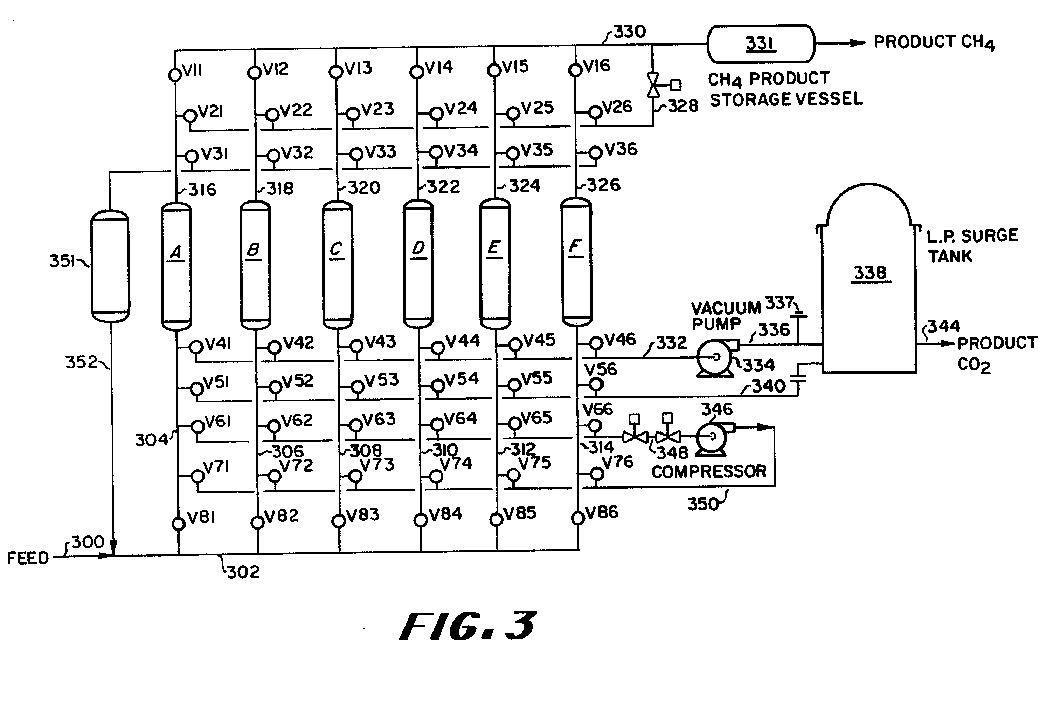

A 6 bed embodiment of the present invention is illustrated in FIG 3. This process includes a two-stage depressurization sequence following the high pressure rinse step. Therefore, the cycle sequence for the 6 bed configuration includes an adsorption step, a high pressure rinse step, a first step of desorptive depressurization, a second step of desorptive depressurization, an evacuation step and a repressurization step. The cycle sequence will be described for bed A, which is in parallel with beds B, C, D, E and F wherein feed is delivered to the various beds through feed lines 304, 306, 308, 310, 312 and 314 and primary component effluent is recovered through lines 316, 318, 320, 322, 324 and 326 of the respective beds. With regard to bed A, a feed gas containing methane and carbon dioxide, the primary and secondary components respectively, is introduced at a pressure in the range of approximately 50-500 psia in line 300 from a pretreatment zone. The feed gas is mixed with a recycle gas from line 352. The combined stream is introduced through manifold 302 into line 304 through open valve 81. The gas is introduced into bed A which is packed with a molecular sieve 13X, or other adsorbents, which is selectively adsorptive to carbon dioxide over methane. A methane primary component product is removed in line 316 and delivered through open valve 11 into manifold 330 and into the methane product storage vessel 331. The methane is removed as product and a portion is used for repressurizing one of the other parallel beds which is going through the regeneration sequence. When the carbon dioxide adsorption wave front approaches the downstream end of adsorptive bed A, the adsorption step is terminated.

With the appropriate changing of valves, the high pressure rinse step is then initiated in bed A whereby secondary component or carbon dioxide from another bed is removed through line 348, compressed in compressor 346 and delivered to bed A through line 350, open valve 71 and feed line 304. This purges bed A cocurrently of any void gas and residual methane in the interstitial spaces between the molecular sieve or other adsorbent packing material along with any co-adsorbed methane. The effluent from bed A during the rinse step has a compositional quality approaching that of the feed gas in line 300 and is therefore passed through valve 31 into vessel 351 and line 352 for recycle and admixture with the feed gas for separation in another of the parallel set of adsorptive beds. Since the carbon dioxide concentration varies over the rinse step, one may use a mixing tank 351 to insure that the rinse effluent has a consistent composition when recycled to feed 300. The high pressure rinse is terminated when the carbon dioxide wave front approaches the downstream end of bed A and all void gas is removed from said bed.

Again, with the appropriate switching of valves, bed A is subjected to the first stage of depressurization wherein the high purity carbon dioxide saturating bed A is removed countercurrently from a pressure in the range of 50-500 psia, the feed pressure, to a first intermediate pressure level in the range of 20-250 psia. The desorbed carbon dioxide gas passes through open valve 61 and line 348 to be recompressed in compressor 346, before the high pressure carbon dioxide in line 350 is introduced into another bed undergoing the high pressure rinse step. Using this gas cut as rinse saves power in comparison to pressurizing a subsequent gas cut or gas from vessel 338.

At the end of the first depressurization, the valves are again switched and the pressure in bed A is further reduced from a pressure in the range of 20-250 psia to a second intermediate pressure, approximately ambient pressure of 14.7 psia, by opening valve 51 and further countercurrently removing the secondary component or carbon dioxide through line 340 into surge tank 338. The desorbed gas from this second depressurization step is collected in the surge tank 338 and is combined with the desorbed gas from the evacuation step.

After bed A reaches the second intermediate pressure in the preceding step, no intermediate purge or rinse is performed as in the prior art, but the depressurization is immediately followed by an evacuation step. The appropriate valves are switched such that bed A is countercurrently evacuated through open valve 41 and line 332 such that carbon dioxide is removed by vacuum system 334 and delivered into surge tank 338 by line 336. This evacuation is performed down to a pressure of preferably 2.2 psia or 115 torr. The product of the evacuation and second stage of depressurization are combined and removed in line 344 for venting or compression to product standards for export of the carbon dioxide. Alternately some venting can occur through vent 337. Thus in this embodiment of the process of the present invention, evacuated carbon dioxide is not used as carbon dioxide rinse gas as in the previous two configurations.

After the low pressure in the range of 1-2.5 psia is obtained in bed A, appropriate valving is switched such that product methane from another bed in the adsorption sequence is passed in line 328 through now open valve 21 and repressurizes bed A countercurrently through line 316 until a pressure approximating the feed pressure in the range of 50-500 psia is obtained. This countercurrent repressurization forces any residual carbon dioxide to the front end of bed A and completes the regeneration of bed A for return to the adsorption step of the overall cycle sequence. Each other bed, B, C, D, E and F goes through a similar sequence of steps through the entire cycle such that a continuous production of methane product and carbon dioxide by-product are available from the feed gas mixture entering the system. When using the 6 bed configuration of the present invention, an additional step is introduced whereby the desorptive depressurization is conducted in 2 steps wherein the first portion of the depressurized product is used for rinse, while the second portion is used as product. This operation significantly reduces the cost and energy of compression of the carbon dioxide rinse gas. Instead of compressing the carbon dioxide from 14.7 psia to the feed pressure as in the first two embodiments of the present invention, carbon dioxide is compressed only from the recited pressure in the range of 20-250 psia intermediate pressure to the feed gas pressure in the six bed embodiment, thus requiring less energy of compression. The precise operation of the overall sequence of the various valve positions is set forth in Table 4 below and a representative operation sequence for bed A is identified at the base of the Table.

## TABLE 4

| Valve | Time (Min.) | | | | | |
|---|---|---|---|---|---|---|
| | 1-3 | 3-6 | 6-9 | 9-12 | 12-15 | 15-18 |
| 11 | 0 | X | X | X | X | X |
| 12 | X | X | X | X | X | 0 |
| 13 | X | X | X | X | 0 | X |
| 14 | X | X | X | 0 | X | X |
| 15 | X | X | 0 | X | X | X |
| 16 | X | 0 | X | X | X | X |
| 21 | X | X | X | X | X | 0 |
| 22 | X | X | X | X | 0 | X |
| 23 | X | X | X | 0 | X | X |
| 24 | X | X | 0 | X | X | X |
| 25 | X | 0 | X | X | X | X |
| 26 | 0 | X | X | X | X | X |
| 31 | X | 0 | X | X | X | X |
| 32 | 0 | X | X | X | X | X |
| 33 | X | X | X | X | X | 0 |
| 34 | X | X | X | X | 0 | X |
| 35 | X | X | X | 0 | X | X |
| 36 | X | X | 0 | X | X | X |
| 41 | X | X | X | X | 0 | X |
| 42 | X | X | X | 0 | X | X |
| 43 | X | X | 0 | X | X | X |
| 44 | X | 0 | X | X | X | X |
| 45 | 0 | X | X | X | X | X |
| 46 | X | X | X | X | X | 0 |
| 51 | X | X | X | 0 | X | X |
| 52 | X | X | 0 | X | X | X |
| 53 | X | 0 | X | X | X | X |
| 54 | 0 | X | X | X | X | X |
| 55 | X | X | X | X | X | 0 |
| 56 | X | X | X | X | 0 | X |
| 61 | X | X | 0 | X | X | X |
| 62 | X | 0 | X | X | X | X |
| 63 | 0 | X | X | X | X | X |
| 64 | X | X | X | X | X | 0 |
| 65 | X | X | X | X | 0 | X |
| 66 | X | X | X | 0 | X | X |
| 71 | X | 0 | X | X | X | X |
| 72 | 0 | X | X | X | X | X |
| 73 | X | X | X | X | X | 0 |
| 74 | X | X | X | X | 0 | X |
| 75 | X | X | X | 0 | X | X |
| 76 | X | X | 0 | X | X | X |
| 81 | 0 | X | X | X | X | X |
| 82 | X | X | X | X | X | 0 |
| 83 | X | X | X | X | 0 | X |
| 84 | X | X | X | 0 | X | X |
| 85 | X | X | 0 | X | X | X |
| 86 | X | 0 | X | X | X | X |
| Bed A → | Adsorption | H.P. Rinse | Depress I | Depress II | Evacuation | Repress |

The present invention realizes improved recovery of secondary product or carbon dioxide at an unexpected magnitude. This is achieved with a lower overall capital cost for an installation which is capable of operating under the present invention's process sequence. Although the omission of the inert purge or air purge required of the prior art might seem to lead one to a higher recovery of secondary product, there are systems which would not allow for such an omission, such as gas separations of methane and normal butane where the secondary component is relatively more sharply adsorbed than carbon dioxide. Further the magnitude of improvement in secondary product recovery defined as moles of component in final product divided by moles of component in fresh feed, could never have been expected.

With reference to Table 5 below, it can be seen that a comparison of the present invention involving 5 steps in a cycle sequence per bed in contrast to the prior art U.S. Patent 4,077,779 using 6 steps in a cycle sequence per bed, produces the equivalent methane purity and recovery of 99% but an overwhelming improvement is seen in the carbon dioxide recovery, although both the present invention and the prior art maintain a carbon dioxide purity of 99%. The insensitivity of these parameters of methane recovery and purity and carbon dioxide purity along with the heightened achievement in the parameter of carbon dioxide recovery is surprising and is not general to all chemical systems as noted above for the methane/normal butane systems.

A drawback to the method of U.S. Patent 4,077,779, as applied to the separation of methane and carbon dioxide, exists in the amount of carbon dioxide recovered during depressurization versus the amount necessary for high pressure rinse. For operational stability and peak recovery, it is necessary to remove more carbon dioxide during depressurization than is utilized in carbon dioxide high pressure rinse of another bed. As set forth in Table 5 the present invention enjoys a dramatic magnitude of recovery of carbon dioxide while the prior art in U.S. Patent 4,077,779 has a marginal recovery of 5%.

0193716

- 21 -

## TABLE 5

### 2MMSCFD Landfill Gas PSA
### Operating Pressure = 70 psig

| | | $CH_4$ Recovery | $CH_4$ Purity | $CO_2$ Recovery | $CO_2$ Purity | Relative Estimated[a] Power | Relative System Capital |
|---|---|---|---|---|---|---|---|
| 1. | Basic Process (A,C,E,F) | 80% | 99% | 99% | 79% | 1.0 | 1.0 |
| 2. | Present Invention (A,B,C,E,F) | 99% | 99% | 99% | 99% | 1.59 | 1.35 |
| 3. | U.S. Pat. 4,077,779 (A,B,C,D,E,F) | 99% | 99% | 5% | 99% | 1.37 | 1.38 |

KEY: Process Steps

A - Adsorption
B - High Pressure $CO_2$ Rinse
C - Desorption
D - Air/Inert Gas Purge
E - Evacuation
F - Repressurization

[a] Power numbers do not include feed or product compression

Therefore, in designing a commercial installation for a methane/carbon dioxide separation, such as a system for the recovery of methane from landfill gas, the comfortable margin of carbon dioxide recovery provided by the present invention provides a dramatic distinction between it and the process of the prior art in U.S. Patent 4,077,779. At some feed pressures, when 99% methane purity and recovery is required, the 6 step sequence of the prior art requires more secondary component for the high pressure rinse step than is made available through the secondary component depressurization recovery. Such a short fall would begin to effect methane recovery over a series of cycle sequences. Such a shortcoming is not exhibited by the present invention as set forth herein. This dramatic increase in operational capability of the 5 step adsorptive process of the present invention in contrast to the 6 step adsorptive process of U.S. Patent 4,077,779 indicates that the deletion of an inert or air purge in such an adsorptive process leads to an unexpected and significant improvement in the art of adsorptive separation of methane and carbon dioxide gas mixtures.

Another advantage that the current five step process has over the original six step process (U.S. 4,077,779) is the means to increase carbon dioxide recovery. This can be done in the four and five bed systems by venting the bulk of the carbon dioxide produced during the desorption step and collecting only the higher purity carbon dioxide which is collected during the evaluation step. The use of a higher purity carbon dioxide rinse gas will result in improved methane recovery.

The process advantages of the present invention have been documented in laboratory experiments designed to model the adsorptive separation sequence of the proposed commercial scale process of the present invention. Such a demonstrative example is set forth below.

### EXAMPLE

A bench scale unit was run to evaluate the performance of the above mentioned PSA process. A feed gas comprising 42.5% $CO_2$ and 57.5% $CH_4$ at 70 psig pressure and 21°F temperature was fed to a column containing 7 lbs. of 13X zeolite as the adsorbent. A $CH_4$ enriched stream containing 99% $CH_4$ was withdrawn from the column at 69 psig. 0.013 lb. moles of feed gas was passed through the column during the adsorption step. The $CO_2$ rinse step was carried out using a 99% $CO_2$ gas at 72 psig and the effluent was recycled as feed for the adsorption step. The column was then depressurized to ambient pressure level and then evacuated to 95 torr. The effluent was 98.5% $CO_2$, part of which was withdrawn as $CO_2$ product and the other part was recompressed and recycled as $CO_2$ rinse. The column was repressurized to 70 psig after evacuation using part of the 99% $CH_4$ product and the cycle was repeated. The amounts of the $CH_4$ and $CO_2$ products from the system were respectively 0.0074 and 0.0056 lb. moles. The purities of both products were 99%. Thus, the product recovery for both constituents of the feed mixture were 99% (within experimental error).

The present invention has been described above with several specific embodiments, but the scope of the present invention should be ascertained from the claims which follow.

0193716

- 24 -

CLAIMS

1. A process for the adsorptive separation of a gas mixture containing at least a primary gaseous component and a secondary gaseous component in which each component is recovered at high recovery and high purity in a plurality of adsorption beds which are preferentially selective to the adsorption of the secondary component, comprising the steps of:

(a) passing a feed gas mixture into one of the plurality of adsorption beds wherein primary component passes through the bed as a pure product and secondary component is selectively adsorbed on the adsorbent;

(b) discontinuing adsorption and rinsing the adsorption bed with a stream of secondary component to remove any primary component as an effluent which is recycled to the feed to the process;

(c) depressurizing the rinsed adsorption bed to an intermediate pressure countercurrent to said adsorption to at least partially remove the secondary component from said adsorption bed;

(d) following said depressurization of step (c) and without further rinsing, evacuating said adsorption bed countercurrently to a subatmospheric pressure to further remove secondary component from said bed; and

(e) repressurizing the bed to superatmospheric pressure to prepare the bed for another adsorption step.

2. The process of Claim 1 wherein the gas mixture comprises methane as a primary component and carbon dioxide as a secondary component.

3. The process of Claim 1 wherein the separation is performed in four adsorption beds connected in parallel.

4. The process of Claim 1 wherein the separation is performed in five adsorption beds connected in parallel.

5.  The process of Claim 1 wherein the separation is performed in six adsorption beds connected in parallel.

6.  The process of Claim 1 wherein the recovery of primary and secondary component is 95% or above.

7.  The process of Claim 1 wherein the adsorbent is a synthetic or natural zeolite.

8.  The process of Claim 1 wherein the feed gas mixture is landfill gas.

9.  The process of Claim 1 wherein the feed gas mixture is steam reformer off-gas containing hydrogen as a primary component and carbon dioxide as a secondary component.

10.  The process of Claim 1 wherein the feed gas mixture is production gas from an oil field produced by an oxygen fireflood or carbon dioxide flood.

11.  The process of Claim 1 wherein the feed gas mixture is at 50-500 psig.

12.  The process of Claim 1 wherein the feed gas mixture is pretreated to remove water and heavy hydrocarbons.

13.  The process of Claim 1 wherein the rinse of step (b) is preformed cocurrently to the adsorption of step (a).

14.  The process of Claim 1 wherein the repressurization of step (e) is performed countercurrently to the adsorption of step (a).

15. The process of Claim 1 wherein a portion of the secondary component evolved in steps (c) and (d) is used as the rinse gas of step (b).

16. The process of Claim 1 wherein a portion of the secondary component evolved in step (d) is used as the rinse gas in step (b).

17. The process of Claim 1 wherein step (c) is performed in at least two stages.

18. The process of Claim 1 wherein the adsorbent is an activated carbon.

19. The process of Claim 1 wherein the adsorbent is the sodium exchanged form of the X zeolite.

20. The process of Claim 1 wherein the intermediate pressure of step (c) is approximately atmospheric pressure.

21. The process of Claim 17 wherein the secondary component from the first stage of depressurization is used for the rinse of step (b).

3408C
AD108

FIG. I

FIG.2

**FIG. 3**